(19) Europäisches Patentamt
European Patent Office
Office européen des brevets

(11) Publication number: 0 396 827 A1

# (12) EUROPEAN PATENT APPLICATION

(21) Application number: 89304669.8

(22) Date of filing: 09.05.89

(51) Int. Cl.[5]: C07C 323/32, C07C 323/63, A61K 31/135

(43) Date of publication of application: 14.11.90 Bulletin 90/46

(84) Designated Contracting States: AT BE CH DE FR GB IT LI LU NL SE

(71) Applicant: SPOFA Spojené Podniky Pro Zdravotnickou Vyrobu
Husinecká 11 a
Prag 3(CS)

(72) Inventor: Rotiva, Miroslav
7 Bri Capku
Praha 10(CS)
Inventor: Jilek, Jiri
9 Malostranske nam
Praha 1(CS)
Inventor: Valchar, Martin
272 Libcicka
Praha 8(CS)
Inventor: Dlabac, Antonin
2609 Jasminova
Praha 10(CS)
Inventor: Sindelar, Karel
43 Moravska
Praha 2(CS)
Inventor: Metysova, Jirina
9 Duchoslavka
Praha 6(CS)
Inventor: Pomykacek, Josef
19 Gorazdova
Praha 2(CS)
Inventor: Dlohonozkova, Natasa
29/537 Kodanska
Praha 10(CS)

(74) Representative: Fitzpatrick, Alan James et al
Fitzpatricks 4 West Regent Street
Glasgow G2 1RS Scotland(GB)

## (54) 2-Phenylthiobenzylamine dervivatives and acid addition salts thereof.

(57) N,N-Dimethyl-2-(3-hydroxyphenylthio)benzylamine and its acid addition salts possess properties of highly selective inhibitors of 5-hydroxytryptamine re-uptake in mammalian brain structures and are expected to find therapeutic use as potent central antidepressants. This novel compound and its intermediate, the 3-methoxyphenylthio analog, are available by per se known preparative routes, especially by reduction of the corresponding amides with hydride agents, by Leuckart reaction of 2-arylthiobenzaldehydes with dimethylformamide and formic acid, or by demethylation of the respective methoxy compound by means of e.g. pyridine hydrochloride or hydrobromic acid.

EP 0 396 827 A1

## 2-Phenylthiobenzylamine derivatives and acid addition salts thereof

The invention relates to 2-phenylthiobenzylamine derivatives, more particularly to N,N-dimethyl-2-(3-hydroxy-and 3-methoxyphenylthio)benzylamines of formula I

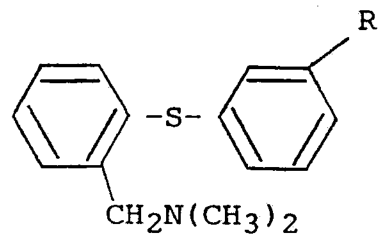

(I)

(compounds IA and IB, respectively) and their addition salts with pharmaceutically acceptable organic or inorganic acids.

Said compound IA in which R is a hydroxy group, and its acid addition salts show properties of highly selective inhibitors of 5-hydroxytryptamine (5-HT) re-uptake in mammalian brain structures and are expected to find therapeutic use as potent central antidepressants.

The above compound was selected as a preferred structure from numerous related new substances on the basis of pharmacological evaluation. Thus, standard biochemical tests on rat hypothalamus with the use of tritium-labelled imipramine (IP) and desipramine (DP) antidepressants (4 nM concentration) proved remarkably higher affinity of compound IA to IP receptors, which indicates selectively serotonergic mechanism of its action (whereas affinity to DP receptors indicates adrenergic route, and conventional nonselective antidepressants have the two affinity values of approximately equal order of magnitude).

The reported selective action of compound IA was verified by direct comparison of $^{3}H$-5-HT (10 nM) and $^{3}H$-noradrenaline (NA, 10 nM) re-uptake in rat brain cortex; again the two sets of values were markedly differentiated in the sense of predominant 5-HT re-uptake inhibition.

Also, standard pharmacological tests of antireserpine activity (on experimental hypothermia, eyelid ptosis and gastric ulceration) and potentiation of yohimbine toxicity in mice are indicative of pronounced antidepressant properties of compound IA.

The substances under study were tested in the form of salts administered p.o. The results are conventionally expressed as medium inhibition concentrations $IC_{50}$ (nM) and effective doses ED (eliciting a first statistically significant response) or medium effective doses $ED_{50}$ (mg per kg); the concentrations and effective doses are calculated as the corresponding amounts of the base.

Acute toxicity was tested in mice p.o. and medium lethal dose $LD_{50}$ is given. Thus, compound IA hydrogen maleate $LD_{50}$ 232 mg/kg. Imipramine and desipramine binding inhibition $IC_{50}$ respectively 4.24 and 12947 nM; 5-HT and NA re-uptake $IC_{50}$ 0.58 and 505543 nM. Reserpine hypothermia ED 10, ptosis 25, ulceration 50 mg/kg. Yohimbine, $ED_{50}$ 54.7 mg/kg.

The compounds of the invention, i.e. N,N-dimethyl-2-(3-hydroxyphenylthio)benzylamine (compound IA) and the respective 3-methoxyphenylthio- analog (compound IB), are available by different preparative routes known per se, such as e.g. reduction of the corresponding amide of the general formula II

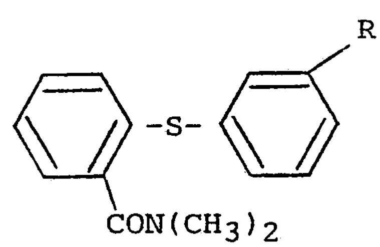

(II)

in which R has the same meaning as in formula I, with hydride agents, e.g. lithium aluminium hydride, or with nascent diborane (from $NaBH_4$ + $BF_3$). The subject methoxy-compound can advantageously be prepared by Leuckart reaction from 2-arylthiobenzaldehydes of the general formula III,

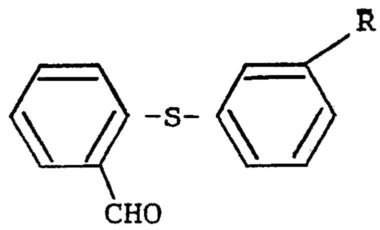

(III)

in which again R is the same as in formula I, with dimethylformamide and formic acid at approximately 170 to 180 °C. The desired hydroxy-compound IA is then obtained by demethylation of the preceding by methods known per se e.g. by heating with pyridine hydrochloride at approximately 180 to 220 °C, boiling with concentrated hydrobromic acid or by reaction with boron tribromide in aprotic polar solvents such as chloroform at ambient temperature.

Starting amides of formula II are novel compounds which can be prepared by current techniques starting with the respective carboxylic acids; these are known materials and are manufactured mostly by reaction of 2-iodobenzoic acid with 3-hydroxy- or 3-methoxythiophenol in boiling aqueous potassium hydroxide solution in the presence of copper. In the case of said hydroxy-intermediate the resulting 2-(3-hydroxyphenylthio)benzoic acid is immediately reacted with dimethylamine, e.g. in the presence of triphenylphosphinetetrachloromethane complex in tetrahydrofuran.

The structure of the novel products was verified using common analytical and spectral methods. For preparing the respective medicinal dosage forms and pharmacological evaluation the base of compound I is advantageously converted into crystalline, more readily water-soluble addition salts with pharmaceutically acceptable organic or inorganic acids.

The invention will now be described further by way of the following non-limitative examples.

Example 1

a) 2-(3-Methoxyphenylthio)benzoyl chloride.

To a stirred solution of 30g of 2-(3-methoxyphenylthio)benzoic acid (Bartl V. et al., Collect. Czech. Chem. Commun. 38, 2301, 1973) in 250 ml of benzene there are added 2 drops of dimethylformamide, 45 g of thionyl chloride is dropped in and the mixture is refluxed for 2 hours. The volatiles are evaporated, the residue is dissolved while hot in 55 ml of cyclohexane and the solution is rapidly filtered and allowed overnight to crystallize to yield 30.4 g (95%) of 2-(3-methoxyphenylthio)benzoyl chloride, m.p. 101 -101.5 °C.

b) N,N-Dimethyl-2-(3-methoxyphenylthio)benzamide.

A solution of the preceding acyl chloride (24.1 g) in 180 ml of benzene is added dropwise at 10 °C under vigorous stirring to 160 ml of 20% aqueous dimethylamine. Stirring is continued for 2 hours at room temperature and the benzenic layer is separated, washed with water, filtered and evaporated under reduced pressure to give an oily residue (23.1 g, 93%) which crystallizes spontaneously on standing, m.p. 42 - 43 °C (petroleum ether).

c) N,N-Dimethyl-2-(3-methoxyphenylthio)benzylamine -Procedure A.

A solution of the preceding benzamide (22.1 g) in 350 ml of ether is added during 40 minutes to a stirred solution of 8.7 g of lithium aluminium hydride in 150 ml of ether. The mixture is refluxed for 6 hours and on cooling it is decomposed by slow addition successively of 9 ml of water, 9 ml of 15% sodium hydroxide solution and 27 ml of water. After stirring for 20 minutes the solid material is filtered off, washed with ether and the filtrate is dried and evaporated to give oily crude product (19.5 g, 93%); its neutralization with a solution of hydrogen chloride in ether affords the crystalline hydrochloride, m.p. 149 - 150 °C (ethanol - ether).

c) N,N-Dimethyl-2-(3-methoxyphenylthio)benzylamine -Procedure B

To a solution of 68.7 g of N,N-dimethyl-2(3-methylphenylthio)-benzamide in 470 ml of tetrahydrofuran are added with stirring under nitrogen 13.1 g of sodium borohydride, and 68.7 g (61 ml) of boron trifluoride etherate are dropped in within 1 hour at 20 to 27 ° C. The mixture is stirred for another hour at room temperature and then refluxed for 3 hours. On cooling it is acidified, under continuous stirring, by slow addition (within 30 minutes) of 190 ml of dilute hydrochloric acid (1 : 1 by vol.). The mixture is refluxed for 3 hours, cooled and made alkaline with 400 ml of a 20% sodium hydroxide solution. The aqueous phase is diluted with 200 ml of water and extracted with 1,2-dichloroethane, the combined organic extracts are dried and evaporated, the residue is dissolved in 40 ml of ethanol, slight excess of ethanolic hydrogen chloride solution and 40 ml of cyclohexane are added, and the solution is inoculated and allowed to crystallize in a refrigerator the yield is 58.9 g (80%) of N,N-dimethyl-2-(3-methoxyphenylthio)benzylamine hydrochloride, m.p. 147.5 - 150 ° C, which is identical with the product of the preceding Procedure A.

Example 2

a) 2-(3-Methoxyphenylthio)benzaldehyde.

A mixture of 20.0 g of 3-methoxythiophenol (Mauthner F., Ber. Dtsch.Chem.Ges. 39, 3596, 1906), 40 ml of dimethylformamide, 20 g of potassium carbonate and 20.1 g of 2-chlorobenzaldehyde is stirred for 5 hours at 90 ° C. After that it is diluted with 200 ml of water and extracted with benzene. Processing of the extract and crystallization of the crude product from 25 ml of methanol affords 28.5 g (82%) of the desired material, m.p. 63.5 - 64.5 ° C.

b) N,N-Dimethyl-2-(3-methoxyphenylthio)benzylamine

A mixture of 28.5 g of 2-(3-methoxyphenylthio)benzaldehyde, 43 g of dimethylformamide and 26.8 g of formic acid is refluxed under stirring for 6.5 hours (bath temperature 180 ° C). On cooling there is added 5% hydrochloric acid (170 ml), the solution is washed with ether, the aqueous acidic portion is made alkaline with 20% sodium hydroxide and the liberated base is separated by extraction with 1,2-dichloroethane. Processing of the extract gives 30.3 g of the crude product, which is then converted into the hydrochloride (27.1 g, 75%), m.p. 149 -150 ° C.

Example 3

N,N-Dimethyl-2-(3-hydroxyphenylthio)benzylamine

A mixture of 214 g of N,N-dimethyl-2-(3-methoxyphenylthio);benzylamine hydrochloride and 850 ml of 47% hydrobromic acid is refluxed under stirring for 8 hours at 120 ° C. On cooling it is poured into 1.85 litres of water and made alkaline to pH 9 by slowly adding 1200 ml of 20% sodium hydroxide solution. Extraction with 2.5 litres of 1,2-dichloroethane and processing of the extract yields 170 g (95%) of the desired crystalline base, m.p. 107 -108 ° C (methanol); hydrogen maleate, m.p. 123 - 124 ° C (ethanol -ether), hydrochloride, m.p. 165 - 166 ° C (ethanol), hydrobromide, m.p. 150 -151 ° C (ethanol - ether).

## Claims

1. 2-Phenylthiobenzylamine derivatives of the general formula I

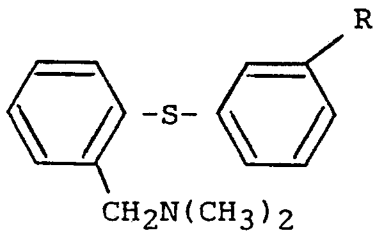

(I)

in which R is a hydroxy or methoxy group, and addition salts thereof with pharmaceutically acceptable organic or inorganic acids.

2. N,N-Dimethyl-2-(3-hydroxyphenylthio)benzylamine and acid addition salts thereof.

3. N,N-Dimethyl-2-(3-methoxyphenylthio)benzylamine and acid addition salts thereof.

4. 2-Phenylthiobenzylamides of the general formula II in which R is a hydroxy or methoxy group.

5. A medicament for use as a central antidepressant comprising a 2-phenylthiobenzylamine derivative as claimed in claim 1 and a pharmaceutically acceptable carrier.

6. Use of a 2-phenylthiobenzylamine derivative as claimed in claim 1 as a central antidepressant.

European Patent Office

# EUROPEAN SEARCH REPORT

Application Number

EP 89 30 4669

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | US-A-4 018 830 (CHRISTY)<br>* Column 4, line 32 - column 5, line 28; examples 5,6; claim 3 *<br>--- | 1-4 | C 07 C 323/32<br>C 07 C 323/63<br>A 61 K 31/135 |
| A | US-A-3 997 540 (MEHTA et al.)<br>* Column 1, line 1 - column 3, line 20; column 5, line 1 - column 6, line 59 *<br>--- | 1-6 | |
| A | US-A-4 208 205 (FAWZI)<br>* Examples; claims 1-7 *<br>----- | 4 | |
| | | | **TECHNICAL FIELDS SEARCHED (Int. Cl.5)** |
| | | | C 07 C 323/00 |

The present search report has been drawn up for all claims

| Place of search | Date of completion of the search | Examiner |
|---|---|---|
| THE HAGUE | 10-01-1990 | HELPS I.M. |

**CATEGORY OF CITED DOCUMENTS**

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)